(19) Europäisches Patentamt
European Patent Office
Office européen des brevets

(11) Veröffentlichungsnummer: **0 229 635**
**A2**

(12) # EUROPÄISCHE PATENTANMELDUNG

(21) Anmeldenummer: **87100133.5**

(22) Anmeldetag: **08.01.87**

(51) Int.Cl.[3]: **C 07 D 498/06**
C 07 D 498/16, C 07 D 471/06
C 07 D 471/16, C 07 C 101/34
A 23 K 1/16, A 61 K 31/535
A 61 K 31/55
//(C07D498/06, 265:00, 221:00),
(C07D498/06, 267:00, 221:00),
(C07D471/06, 241:00, 221:00),
(C07D498/16, 265:00, 221:00, 221:00)

(30) Priorität: **15.01.86 DE 3600891**

(43) Veröffentlichungstag der Anmeldung:
**22.07.87 Patentblatt 87/30**

(84) Benannte Vertragsstaaten:
**AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Schriewer, Michael, Dr.**
**Am Thelen Siefen 1a**
**D-5068 Odenthal(DE)**

(72) Erfinder: **Grohe, Klaus, Dr.**
**Am Wasserturm 10**
**D-5068 Odenthal(DE)**

(72) Erfinder: **Zeiler, Hans-Joachim, Dr.**
**Elsbeekerstrasse 46**
**D-5620 Velbert(DE)**

(72) Erfinder: **Metzger, Karl Georg, Dr.**
**Pahlkestrasse 75**
**D-5600 Wuppertal(DE)**

(54) **1,8-Verbrückte 4-Chinolon-3-carbonsäuren und diese enthaltende Arzneimittel.**

(57) Die Erfindung betrifft ein neues, chemisch eigenartiges Verfahren zur Herstellung von neuen 1,8-verbrückten 4-Chinolon-3-carbonsäuren und Derivaten der Formel (I)

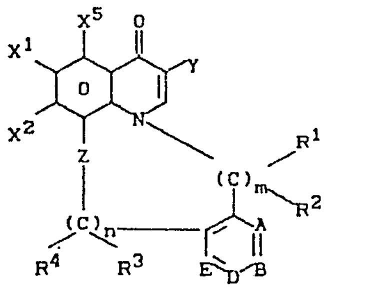

(I)

in welcher Y, $X^1$, $X^2$, $X^5$, Z, $R^1$ bis $R^4$, n sowie die Symbole A, B, D und E die in der Beschreibung angegebene Bedeutung haben, sowie ihre Verwendung als Arzneimittel, insbesondere als antibakterielle Mittel in der Human- und Veterinärmedizin und als Zwischenprodukte für antibakterielle Mittel.

EP 0 229 635 A2

BAYER AKTIENGESELLSCHAFT 5090 Leverkusen, Bayerwerk
Konzernverwaltung RP
Patentabteilung Ad/li-c

# 1,8-Verbrückte 4-Chinolon-3-carbonsäuren und diese enthaltende Arzneimittel

Die Erfindung betrifft ein neues, chemisch eigenartiges Verfahren zur Herstellung von neuen 1,8-verbrückten 4-Chinolon-3-carbonsäuren, sowie ihre Verwendung als Arzneimittel, insbesondere als antibakterielle Mittel in der Human- und Veterinärmedizin und als Zwischenprodukt für antibakterielle Mittel.

In der Europäischen Patentanmeldung 00 04 279 wird die Herstellung von 4-Pyridon-3-carbonsäuren durch Umsetzung von tautomeriefähigen Enaminen mit o-Halogen-aryl-carbonsäurehalogeniden und anschließende Cyclisierung in Gegenwart einer Base beschrieben.

Es wurden nun neue 1,8-verbrückte 4-Chinolon-3-carbonsäuren und Derivate der Formel I

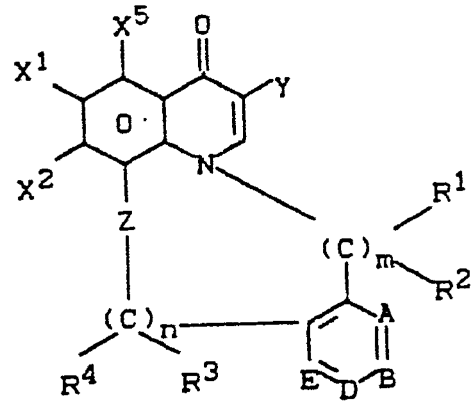

(I)

in welcher

Y eine Carboxylgruppe, eine Nitrilgruppe, eine Estergruppe $-COOR^5$ oder Säureamidgruppe $-CONR^6R^7$ darstellt, wobei $R^5$ für $C_1$-$C_4$-Alkyl steht, $R^6$ und $R^7$ für Wasserstoff oder $C_1$-$C_4$-Alkyl stehen und $R^6$ außerdem gegebenenfalls Phenyl sein kann,

$X^1$ für Wasserstoff, Nitro, Alkyl mit 1-3 Kohlenstoffatomen und Halogen, bevorzugt Fluor steht,

$X^2$ Halogen bevorzugt Fluor oder Chlor, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff oder einen Aminrest $NR^8$ steht, wobei $R^8$ Wasserstoff oder einen gegebenenfalls durch Halogen, Trifluormethyl, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen oder einen gegebenenfalls durch Halogen, eine Nitrogruppe, eine Alkylgruppe mit 1-3 Kohlenstoffatomen substituierten Phenylrest bedeutet, sowie ferner einen Acylrest $R^9CO$- bzw. $R^{10}SO_2$- darstellt, wobei $R^9$ und $R^{10}$ Alkylreste mit 1-6 Kohlenstoffatomen oder gegebenenfalls substituierte Phenylreste darstellen, sowie ein

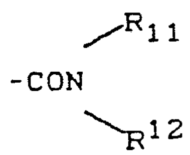 oder 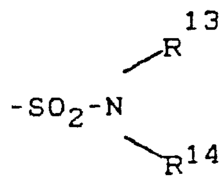 -Rest sein kann

wobei die Reste $R^{11}$ bis $R^{14}$ Wasserstoff, Alkyl mit 1-6 Kohlenstoffatomen oder einen gegebenenfalls substituierten Phenylrest darstellen,

$R^1$, $R^2$, $R^3$ und $R^4$ für Wasserstoff und Alkylgruppen mit 1-3 Kohlenstoffatomen oder für gegebenenfalls durch Halogen, Nitro, Alkyl mit bis zu 3 Kohlenstoffatomen substituierte Phenylreste stehen, sowie

n und m jeweils 0 oder 1 bedeuten,

die Symbole A, B, D und E für $C-R^{15}$ oder bis zu drei dieser Symbole für Stickstoff stehen, wobei

$R^{15}$ für Wasserstoff, Alkyl-, Alkoxy-, Alkylmercapto mit jeweils 1-3 Kohlenstoffatomen, Halogen, Nitro, Trifluormethyl, Cyano, durch $C_1$-$C_4$-Alkyl verestertes Carboxyl, Benzyl oder Phenyl, die jeweils durch $C_1$-$C_3$-Alkyl, Nitro oder Halogen substituiert sein können, steht, mit wertvollen pharmazeutischen Eigenschaften gefunden.

Physiologisch verträgliche Salze bzw. Prodrug-Formen gehören ebenfalls zur Erfindung.

Die erfindungsgemäßen Verbindungen werden erhalten, wenn man die Enamine der Formel II

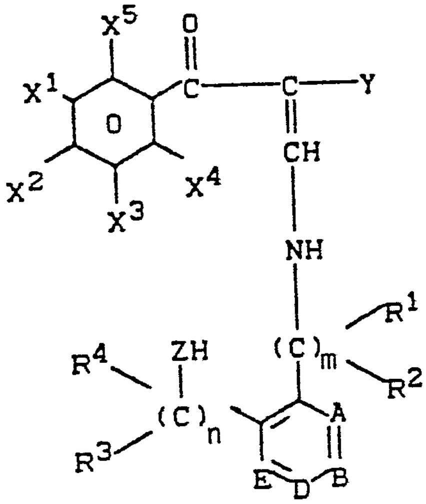

(II)

in welcher

$X^1$, $X^2$, $X^5$, $R^1$-$R^4$, A, B, D, E, Y, Z, m und n die oben angegebene Bedeutung haben und

$X^3$ für Halogen, bevorzugt Fluor oder Chlor sowie die Nitrogruppe steht,

$X^4$ Halogen, bevorzugt Fluor oder Chlor, eine Nitrogruppe, eine Alkoxy-, Alkylmercapto- oder Alkylsulfonylgruppe mit jeweils 1-3 Kohlenstoffatomen sowie eine Arylsulfonylgruppe bedeutet,

in einer ersten Reaktionsstufe in einem wasserfreien, aprotischen Lösungsmittel in Gegenwart von 1 Äquivalent einer Base zu den 4-Chinolon-3-carbonsäurederivaten der Formel III

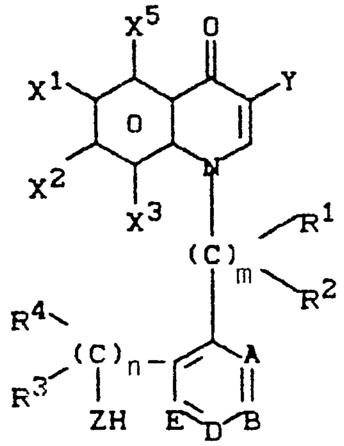

(III)

umsetzt und in einer zweiten Reaktionsstufe in Gegenwart eines weiteren Äquivalents einer Base die Zweitcyclisierung zu den 1,8-verbrückten 4-Chinolon-3-carbonsäurederivaten der Formel I vornimmt, sowie gegebenenfalls die Gruppe Y in an sich bekannter Weise in die Carboxylgruppe und gegebenenfalls diese in ihre Alkali-, Erdalkali- oder Silbersalze überführt.

Besonders vorteilhaft ist die Umsetzung der Enamine II mit 2 Äquivalenten einer Base, ohne Zwischenisolierung von III, in einer sogenannten Eintopfreaktion zu den 1,8-verbrückten 4-Chinolon-3-carbonsäurederivaten I.

Es ist als ausgesprochen überraschend zu bezeichnen, daß die 1,8-Überbrückung in der zweiten Reaktionsstufe des erfindungsgemäßen Verfahrens unter Beteiligung einer durch die meta-ständige Carboxylgruppe mesomer nicht aktivierten Abgangsgruppe $X^3$ erfolgt.

Verwendet man den Enaminoester 1 als Ausgangsstoff, so kann der Reaktionsablauf durch das folgende Formelschema wiedergegeben werden (1 ⟶ 1' ⟶ 1" ⟶ 1'"):

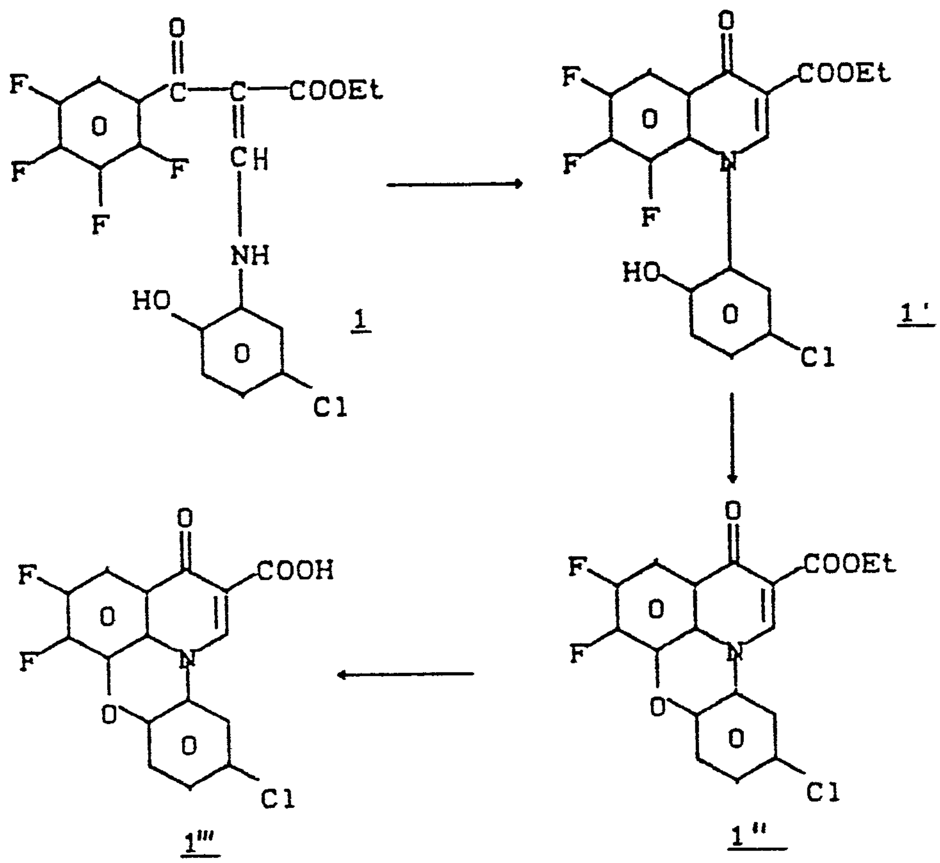

1 1' 1" 1'"

Die als Ausgangsstoffe verwendeten Enamine II sind neu und können durch Umsetzung der entsprechenden Enolether IV mit den primären Aminen V hergestellt werden, wobei $X^1$-$X^5$, $R^1$-$R^4$, Y, Z, A, B, D, E, n und m die oben angegebene Bedeutung haben.

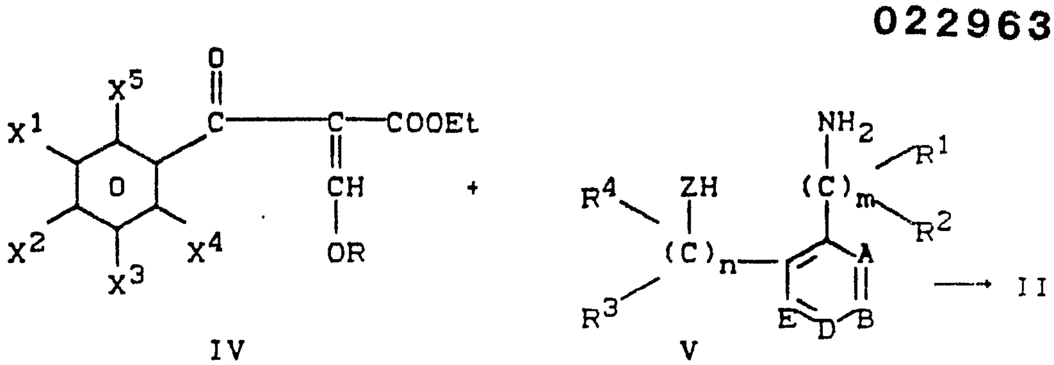

IV V

$R=CH_3$, $C_2H_5$, $n-C_3H_7$

Die Enolether IV sind bekannt oder können gemäß folgendem allgemeinen Reaktionsschema hergestellt werden:

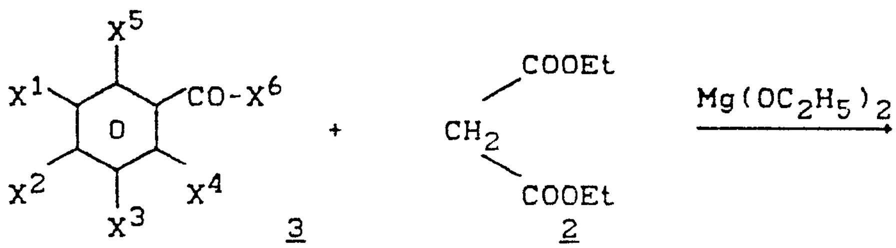


3 2

$X^6$ = F, Cl, Br

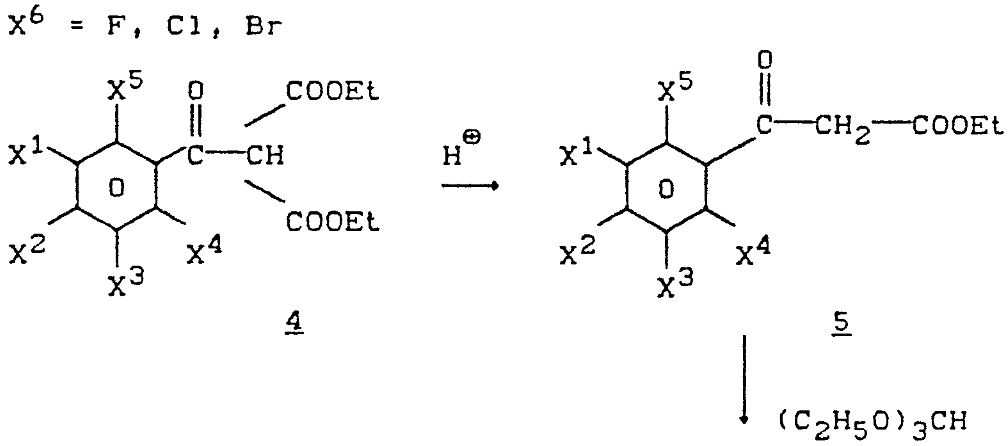


4 5

IV

Danach wird Malonsäurediethylester 2 in Gegenwart von Magnesiumethylat mit dem entsprechenden Benzoylhalogenid 3 zum Acylmalonester 4 acyliert (Organicum, 3. Aufl. 1964, S. 438).

Durch partielle Verseifung und Decarboxylierung von 4 in wäßrigem Medium mit katalytischen Mengen Schwefelsäure oder 4-Toluolsulfonsäure erhält man in guter Ausbeute den Acylessigsäureethylester 5, der mit Orthoameisensäuretriethylester/Acetanhydrid in den 2-Benzoyl-3-ethoxy-acrylsäureethylester (IV, $R=C_2H_5$) übergeht. Die Umsetzung von (IV) mit den Aminen (V) in einem Lösungsmittel wie z. B. Methylenchlorid, einem Alkohol, Chloroform, Cyclohexan oder Toluol führt in leicht exothermer Reaktion zu den gewünschten Zwischenprodukten (II).

Die Cyclisierungsreaktionen (II) $\longrightarrow$ (III), (III) $\longrightarrow$ (I) und (II) $\longrightarrow$ (I) werden in einem Temperaturbereich von etwa 60-300° C, bevorzugt 80-180° C durchgeführt.

Als Verdünnungsmittel können Dioxan, Dimethylsulfoxid, N-Methylpyrrolidon, Sulfolan, Hexamethylphosphorsäuretriamid und bevorzugt N,N-Dimethylformamid verwendet werden.

Die Cyclokondensationen können bei Normaldruck, aber auch bei erhöhtem Druck durchgeführt werden. Im allgemeinen arbeitet man bei Drucken zwischen etwa 1 und etwa 100 bar, vorzugsweise 1 und 10 bar.

Als Säurebinder kommen für die Cyclisierungsreaktionen (II) $\longrightarrow$ (III), (III) $\longrightarrow$ (I) und (II) $\longrightarrow$ (I) Kalium-tert.-butanolat.
1,4-Diaza-bicyclo[2,2,2]-octan(DABCO), 1,8-Diaza-bicyclo[5,4,0]-undec-7-en(DBU), Butyl-lithium, Lithium-phenyl,

Phenylmagnesiumbromid, Natriummethylat, Natriumhydrid, Natrium- oder Kaliumcarbonat in Betracht. Besonders bevorzugt werden Kalium- oder Natriumfluorid, wenn Fluorwasserstoff abgespalten werden muß.

Für die Primärcyclisierung (II) ⟶ (III) und die Zweitcyclisierung (III) ⟶ (I) wird im allgemeinen jeweils 1 Äquivalent Base verwendet. Werden beide Cyclisierungsreaktionen in der "Eintopf-Reaktion" (II) ⟶ (I) zusammengefaßt, so müssen 2 Äquivalente der oben angegebenen Basen eingesetzt werden. Es kann vorteilhaft sein, bei den Cyclokondensationen (III) ⟶ (I) und (II) ⟶ (I) einen Überschuß von 10 Mol-% Base einzusetzen.

Die im letzten Schritt erfolgende Hydrolyse der Ester, Nitrile und Amide (I) zu den entsprechenden Carbonsäuren kann unter den üblichen bekannten sauren oder basischen Bedingungen erfolgen.

Die als Ausgangsstoffe für diesen Syntheseweg verwendete 2,3,4,5-Tetrafluorbenzoylchlorid und Pentafluorbenzoylchlorid sind bekannt.

3,5-Dichlor-2,4-difluor-benzoylfluorid (Siedepunkt 97° / 20 mbar; $n_D^{20}$ = 1,5148) und 5-Chlor-2,3,4-trifluorbenzoylfluorid (Siedepunkt 66-70° /20 mbar; $n_D^{20}$ = 1,4764) erhält man nebeneinander beim Erhitzen von Tetrachlorbenzoylchlorid mit Kaliumfluorid in Sulfolan auf erhöhte Temperaturen:

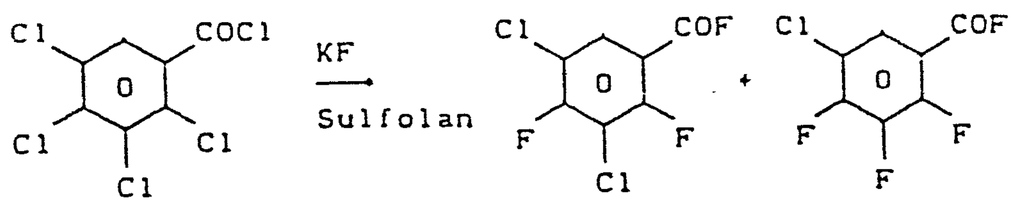


Die Chlorierung von 2,4,5-Trifluorbenzoesäure in Chlorsulfonsäure führt zur 3-Chlor-2,4,5-trifluorbenzoesäure, die als Rohprodukt mit Thionylchlorid zum 3-Chlor-2,4,5-trifluorbenzoylchlorid (Siedepunkt 94° /18 mbar; $n_D^{20}$ = 1,5164) umgesetzt wird:

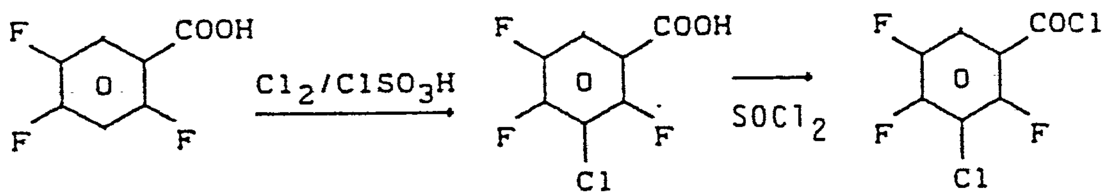


Das 2,4-Dichlor-5-fluor-3-nitro-benzoylchlorid wird durch Nitrierung der bekannten 2,4-Dichlor-5-fluor-benzoesäure zur 2,4-Dichlor-5-fluor-3-nitro-benzoesäure und deren Umsetzung mit Thionylchlorid erhalten.

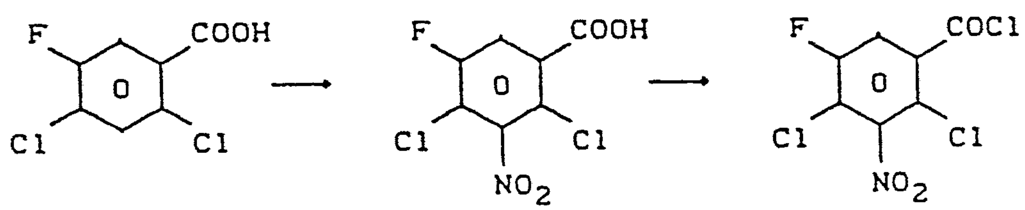

Die als Ausgangsstoffe verwendeten Amine der Formel V sind bekannt. Als Beispiele seien genannt:

0229635

2-Aminophenol, 2-Amino-4-chlor-phenol, 2-Amino-4-fluor-phenol, 2-Amino-5-fluor-phenol, 2-Amino-4-trifluormethyl-phenol, 2-Amino-4nitro-phenol, 2-Amino-3-hydroxy-pyridin, N-Acetyl-phenylen-diamin, 2-Hydroxy-benzylamin, 2-Aminobenzyl-alkohol, 2-Hydroxymethyl-benzylamin.

Die neuen Wirkstoffe besitzen bakterizide Eigenschaften und können außerdem als Zwischenprodukte für hochwirksame antibakterielle Mittel dienen.

Beispiel für eine erfindungsgemäße Tablette

Jede Tablette enthält:

| | |
|---|---|
| Verbindung des Beispiels 28 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Macrogol 4000 rec. INN Polyethylenglykole (DAB) | 2,0 mg |
| Titan-(IV)-oxid | 2,0 mg |
| | 10,0 mg |

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die

folgenden Erreger oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die folgenden Beispiele erläutern die Erfindung:

Herstellung der Ausgangsverbindungen:

Beispiel A

2-(2,4-Dichlor-5-fluor-3-nitro-benzoyl)-3-ethoxy-acrylsäureethylester

a) 2,4-Dichlor-5-fluor-3-nitro-benzoesäure

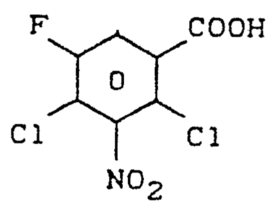

Unter Eiskühlung und Rühren werden 34 ml konz. Schwefelsäure tropfenweise mit 40 ml konz. Salpetersäure versetzt. In dieses Nitriergemisch trägt man portionsweise 20,9 g 2,4-Dichlor-5-fluorbenzoesäure ein, wobei die Temperatur auf 45-50° C steigt. Dann wird noch 3 Stunden auf 90-100° C erhitzt, das auf Raumtemperatur abgekühlte Gemisch auf 350 ml Eiswasser gegossen, der Niederschlag abgesaugt und mit Wasser gewaschen. Das feuchte Rohprodukt wird in 30 ml Methanol heiß gelöst und die Lösung mit 150 ml $H_2O$ versetzt. Der Niederschlag wird kalt abgesaugt, mit $CH_3OH/H_2O$ gewaschen und im Vakuum bei 80° C getrocknet. Es werden 21,2 g rohe 2,4-Dichlor-5-fluor-3-nitro-benzoesäure erhalten. Sie ist genügend rein für

die weiteren Umsetzungen. Eine Probe aus Toluol/Petrolether umkristallisiert liefert Kristalle vom Schmelzpunkt 192° C.

b) 2,4-Dichlor-5-fluor-3-nitro-benzoylchlorid

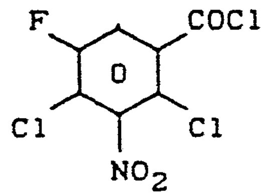

106,6 g 2,4-Dichlor-5-fluor-3-nitro-benzoesäure werden mit 250 ml Thionylchlorid 2 Stunden unter Rückfluß zum Sieden erhitzt. Das überschüssige Thionylchlorid wird dann bei Normaldruck abdestilliert und der Rückstand im Feinvak. fraktioniert. Bei 110-115° C/0,08-0,09 mbar gehen 104,7 g 2,4-Dichlor-5-fluor-3-nitro-benzoyl-chlorid über. Beim Stehen bilden sich Kristalle vom Schmelzpunkt 35-37° C.

c) (2,4-Dichlor-5-fluor-3-nitro-benzoyl)-essigsäureethylester

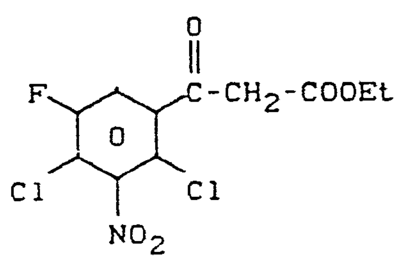

10,1 g Magnesiumspäne werden in 21 ml Ethanol mit 2,1 g Tetrachlormethan versetzt und nach Beginn der Wasserstoff-Entwicklung ein Gemisch aus 66,6 g Malonsäurediethylester, 40 ml Ethanol und 150 ml Toluol bei 50-60°C tropfenweise zugefügt. Man rührt 1 Stunde bei dieser Temperatur nach, kühlt auf -5 bis -10°C und tropft langsam eine Lösung von 109,2 g 2,4-Dichlor-5-fluor-3-nitro-benzoylchlorid in 50 ml Toluol zu. Danach wird 1 Stunde bei 0° gerührt, über Nacht auf Raumtemperatur gebracht und noch 2 Stunden auf 40-50°C erwärmt. Das Reaktionsgemisch wird unter Eiskühlung mit einem Gemisch aus 160 ml Wasser und 10,4 ml konzentrierter Schwefelsäure versetzt und die organische Phase abgetrennt. Die wäßrige Phase wird mit Toluol extrahiert und der vereinigte organische Extrakt mit gesättigter Natriumchlorid-Lösung gewaschen, mit Natriumsulfat getrocknet und das Lösungsmittel abgezogen. Man erhält 144,5 g (2,4-Dichlor-5-fluor-3-nitro-benzoyl)-malonsäurediethylester als Rohprodukt. Dieses wird nach Zugabe von 200 ml Wasser und 0,6 g 4-Toluolsulfonsäure 3 Stunden unter Rückfluß erhitzt, die Mischung mit Methylenchlorid extrahiert, mit Natriumsulfat getrocknet und das Lösungsmittel im Vakuum abdestilliert. Es werden 118 g substituierter Benzoylessigester als Rohprodukt erhalten. Er besitzt eine für die weiteren Umsetzungen genügende Reinheit.

d) 2-(2,4-Dichlor-5-fluor-3-nitro-benzoyl)-3-ethoxy-acryl-säureethylester

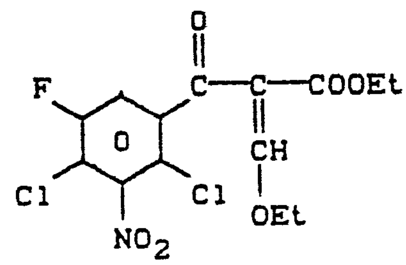

244,8 g (2,4-Dichlor-5-fluor-3-nitro-benzoyl)-essigsäure-ethylester werden mit 166 g Orthoameisensäuretriethylester und 185 g Essigsäureanhydrid 3 Stunden auf 150-160$^{0}$C erhitzt. Man engt dann im Vakuum ein und erhält 270 g Benzoyl-ethoxy-acrylsäureethylester als öligen Rückstand.

Beispiel B

2-(2,3,4,5-Tetrafluor-benzoyl)-3-methoxy-acrylsäurenitril

a) 2,3,4,5-Tetrafluorphenyl-2-chlorvinyl-keton

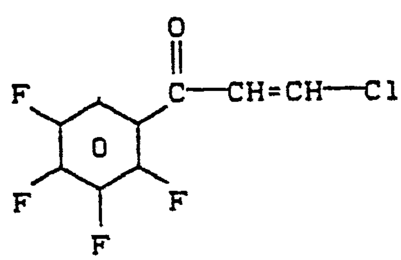

Eine Suspension von 36,5 g wasserfreiem Aluminiumchlorid in 100 ml 1,2-Dichlorethan wird unter Eiskühlung und Rühren tropfenweise mit 53,2 g 2,3,4,5-Tetrafluorbenzoylchlorid bei 0-10$^{0}$C versetzt. Dann

wird bei 40-50° C 7 Stunden Acetylen eingeleitet und auf Eis gegossen. Die Phasen werden getrennt, mit Methylenchlorid nachextrahiert, mit Wasser gewaschen, mit Natriumsulfat getrocknet. Das Lösungsmittel wird im Vakuum abdestilliert und der Rückstand im Hochvakuum destilliert. Bei 92-105° C/1,5 mbar gehen 49,2 g 2,3,4,5-Tetrafluorphenyl-2-chlorvinyl-Keton über.

b) 5-(2,3,4,5-Tetrafluor)-isoxazol

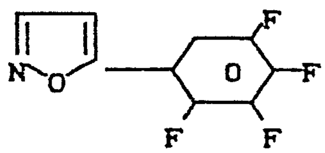

49,2 g 2,3,4,5-Tetrafluorphenyl-2-chlorvinyl-Keton und 14,9 g Hydroxylaminhydrochlorid werden in 100 ml Methanol 6 Stunden refluxiert. Das Lösungsmittel wird im Vakuum abdestilliert, der Rückstand in Methylenchlorid/Wasser aufgenommen, mit Wasser gewaschen, mit Natriumsulfat getrocknet und das Lösungsmittel im Vakuum abgezogen. Es werden 42 g 5-(2,3,4,5-Tetrafluor)-isoxazol als Rohprodukt erhalten.

c) 2,3,4,5-Tetrafluorbenzoylacetonitril

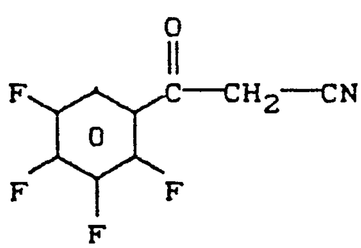

Eine Lösung von 4,5 g Natrium in 150 ml Methanol wird unter Eiskühlung und Rühren tropfenweise mit 41,8 g rohem 5-(2,3,4,5-Tetrafluor)-isoxazol in 60 ml Methanol versetzt. Man rührt 30 Minuten bei Raumtemperatur und erhitzt dann 1 Stunde unter Rückfluß zum Sieden. Das Lösungsmittel wird im Vakuum abdestilliert, der Rückstand in Wasser/Methylenchlorid gelöst, die wäßrige Lösung nochmals mit Methylenchlorid extrahiert und anschließend die Wasser-Phase mit 10%iger Salzsäure auf pH = 3-4 angesäuert. Man extrahiert mit Methylenchlorid, trocknet mit Natriumsulfat und zieht das Lösungsmittel im Vakuum ab. Der Rückstand wird aus wenig Methanol umkristallisiert. Es werden 16,5 g 2,3,4,5-Tetrafluorbenzoyl-acetonitril vom Schmelzpunkt 55-57°C erhalten.

d) 2-(2,3,4,5-Tetrafluorbenzoyl)-3-methoxy-acrylsäurenitril

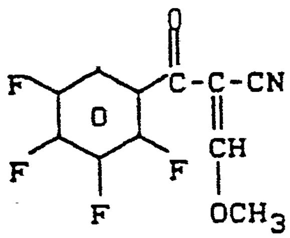

Ein Gemisch von 16 g 2,3,4,5-Tetrafluorbenzoyl-acetonitril, 15,6 g o-Ameisensäuretrimethylester und 18,8 g Acetanhydrid wird 3 Stunden auf 150°C (Badtemperatur) erhitzt. Das Lösungsmittel-Gemisch wird im Wasserstrahlvakuum und zuletzt im Hochvakuum bei einer Badtemperatur von 120°C vollständig abdestilliert. Es werden 18,2 g 2-(2,3,4,5-Tetrafluorbenzoyl)-3-methoxy-acrylsäurenitril als dunkelbraunes Oel erhalten.

Beispiel 1

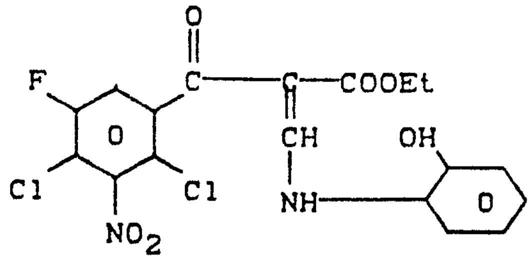

19,0 g 2-(2,4-Dichlor-5-fluor-3-nitro-benzoyl)-3-ethoxyacrylsäureethylester werden in 20 ml Ethanol vorgelegt. Dazu wird eine Suspension von 6 g 2-Aminophenol in 40 ml EtOH gegeben. Man läßt eine Stunde bei Raumtemperatur rühren und isoliert den ausgefallenen Feststoff.

Ausbeute: 17,8 g 2-(2,4-Dichlor-5-fluor-3-nitro-benzoyl)-3-(2-hydroxyphenyl-amino)acrylsäureethylester

Schmelzpunkt: 250-2° (Z)

Analog Beispiel 1 werden folgende Aminoacrylester erhalten (Tabelle 1):

Tabelle 1 Aminoacrylester der Formel II
$R^1$, $R^2$, $R^3$, $R^4$ = H

| Beispiel | $X^1$ | $X^2$ | $X^3$ | $X^4$ | $X^5$ | Z | A | B | D | E | n | m | Y | Schmelzpunkt |
|---|---|---|---|---|---|---|---|---|---|---|---|---|---|---|
| 2 | F | F | F | F | H | O | CH | CH | CH | CH | 0 | 0 | COOEt | 198 - 200° |
| 3 | F | Cl | $NO_2$ | Cl | H | O | CH | CH | CF | CH | 0 | 0 | COOEt | 230 - 2° (Z) |
| 4 | F | F | F | F | H | O | CH | CH | CF | CH | 0 | 0 | COOEt | 206 - 8° |
| 5 | F | F | F | F | H | O | N | CH | CH | CH | 0 | 0 | COOEt | 200 - 1° |
| 6 | F | Cl | $NO_2$ | Cl | H | O | CH | C-$CF_3$ | CH | CH | 0 | 0 | COOEt | 200 - 2° |
| 7 | F | F | F | F | H | O | CH | C-$NO_2$ | CH | CH | 0 | 0 | COOEt | 226 - 8° (Z) |
| 8 | F | Cl | $NO_2$ | Cl | H | O | CH | C-Cl | CH | CH | 0 | 0 | COOEt | 170 - 2° |
| 9 | F | Me | $NO_2$ | Cl | H | O | CH | CH | CH | CH | 0 | 0 | COOEt | 216 - 18° |
| 10 | F | Cl | $NO_2$ | Cl | H | NAc | CH | CH | CH | CH | 0 | 0 | COOEt | 190 - 2° |
| 11 | F | Cl | $NO_2$ | Cl | H | O | CH | CH | CH | CH | 1 | 0 | COOEt | 98 - 100° |
| 12 | F | F | F | F | H | O | CH | CH | CH | CH | 1 | 0 | COOEt | 127 - 29° |
| 13 | F | F | F | F | H | O | CH | CH | CH | CH | 0 | 1 | COOEt | 128 - 30° |
| 14 | F | F | F | F | H | O | C-OH | CH | CH | CH | 0 | 0 | COOEt | 264 - 6° (Z) |

Beispiel 15

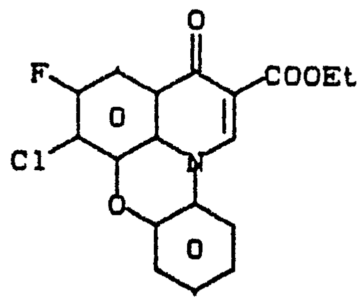

17,8 g Produkt aus Beispiel 1 und 8,2 g $K_2CO_3$ werden in 75 ml DMF 3 Stunden auf 140-45° erhitzt. Nach Abkühlen auf Raumtemperatur wird in Wasser gegossen. Der ausgefallene Feststoff wird abgenutscht und bei 110° getrocknet.
Ausbeute: 12,0 g 1-Chlor-2-fluor-4-oxo-4H-chino[2,3,4i,j]-[1,4]-benzoxazin-5-carbonsäureethylester
Schmelzpunkt 200-2° (Z).

Analog Beispiel 15 werden folgende Chinoloncarbonsäureester erhalten (Tabelle 2):

Tabelle 2 Chinoloncarbonsäureester der Formel I

$R^1$, $R^2$, $R^3$, $R^4$ = H

| Beispiel | $X^1$ | $X^2$ | $X^5$ | Z | A | B | D | E | n | m | Y | Schmelzpunkt |
|---|---|---|---|---|---|---|---|---|---|---|---|---|
| 16 | F | F | H | O | CH | CH | CH | CH | 0 | 0 | COOEt | 214° |
| 17 | F | F | H | O | CH | CH | CF | CH | 0 | 0 | COOEt | 239 - 40° |
| 18 | F | Cl | H | O | CH | CH | CF | CH | 0 | 0 | COOEt | 208 - 10° |
| 19 | F | F | H | O | N | CH | CH | CH | 0 | 0 | COOEt | 188 - 90° (Z) |
| 20 | F | Cl | H | O | CH | $C-CF_3$ | CH | CH | 0 | 0 | COOEt | 238 - 40° (Z) |
| 21 | F | F | H | O | CH | $C-NO_2$ | CH | CH | 0 | 0 | COOEt | 286 - 88° (Z) |
| 22 | F | Me | H | O | CH | CH | CH | CH | 0 | 0 | COOEt | 163 - 66° |
| 23 | F | Cl | H | NH | CH | CH | CH | CH | 0 | 0 | COOEt | 265° (Z) |
| 24 | F | F | H | O | CH | CH | CH | CH | 1 | 0 | COOEt | 230 - 2° |
| 25 | F | Cl | H | O | CH | CH | CH | CH | 1 | 0 | COOEt | 265 - 67° |
| 26 | F | F | H | O | CH | CH | CH | CH | 0 | 1 | COOEt | 230 - 2° |
| 27 | F | Cl | H | O | CH | C-Cl | CH | CH | 0 | 0 | COOEt | 190° (Z) |

Beispiel 28

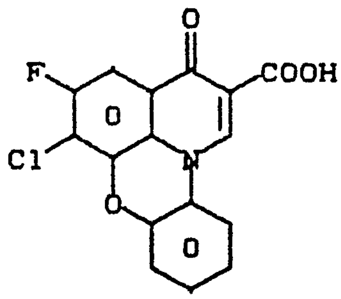

12,0 g Produkt aus Beispiel 15 werden zusammen mit 38 ml Essigsäure, 30 ml Wasser und 3,6 ml konz. Schwefelsäure 2 Stunden gekocht. Nach Abkühlung auf Raumtemperatur wird mit Wasser verdünnt. Der ausgefallene Feststoff wird isoliert.

Ausbeute: 10,0 g.

1-Chlor-2-fluor-4-oxo-4H-chino[2,3,4,i,j][1,4]benzoxazin-5-carbonsäureethylester

Schmelzpunkt: >300° C.

Analog Beispiel 28 wurden folgende Chinoloncarbonsäuren erhalten (Tabelle 3):

Tabelle 3 Chinoloncarbonsäuren der Formel I

$R^1$, $R^2$, $R^3$, $R^4$ = H

| Beispiel | $X^1$ | $X^2$ | $X^5$ | Z | A | B | D | E | n | m | Y | Schmelzpunkt |
|---|---|---|---|---|---|---|---|---|---|---|---|---|
| 29 | F | F | H | O | CH | CH | CH | CH | 0 | 0 | COOH | >300° C |
| 30 | F | F | H | O | CH | CH | CF | CH | 0 | 0 | COOH | 284 - 88° |
| 31 | F | F | H | O | N | CH | CH | CH | 0 | 0 | COOH | >300° |
| 32 | F | Cl | H | O | CH | $C-CF_3$ | CH | CH | 0 | 0 | COOH | >300° |
| 33 | F | F | H | O | CH | $C-NO_2$ | CH | CH | 0 | 0 | COOH | >300° |
| 34 | F | Me | H | O | CH | CH | CH | CH | 0 | 0 | COOH | >300° |
| 35 | F | Cl | H | NH | CH | CH | CH | CH | 0 | 0 | COOH | >300° |
| 36 | F | F | H | O | CH | CH | CH | CH | 1 | 0 | COOH | >300° |
| 37 | F | F | H | O | CH | CH | CH | CH | 0 | 1 | COOH | >300° |

## Patentansprüche

1. 1,8-Verbrückte 4-Chinolon-3-carbonsäuren und Derivate der Formel I

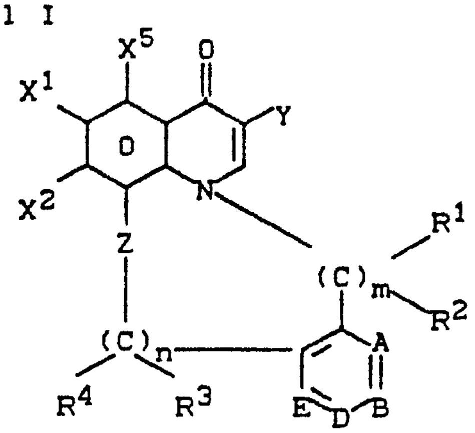

(I)

in welcher

Y eine Carboxylgruppe, eine Nitrilgruppe, eine Estergruppe $-COOR^5$ oder Säureamidgruppe $-CONR^6R^7$ darstellt, wobei $R^5$ für $C_1$-$C_4$-Alkyl steht, $R^6$ und $R^7$ für Wasserstoff oder $C_1$-$C_4$-Alkyl stehen und $R^6$ außerdem gegebenenfalls Phenyl sein kann,

$X^1$ für Wasserstoff, Nitro, Alkyl mit 1-3 Kohlenstoffatomen und Halogen steht,

$X^2$ Halogen, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff oder einen Aminrest $NR^8$ steht, wobei $R^8$ Wasserstoff oder einen gegebenenfalls durch Halogen, Trifluormethyl, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen oder einen gegebenenfalls durch Halogen, eine Nitrogruppe, eine Alkylgruppe mit 1-3 Kohlenstoffatomen substituierten Phenylrest bedeutet, sowie ferner einen Acylrest $R^9CO$- bzw. $R^{10}SO_2$- darstellt, wobei $R^9$ und $R^{10}$ Alkylreste mit 1-6 Kohlenstoffatomen oder gegebenenfalls substituierte Phenylreste darstellen, sowie ein

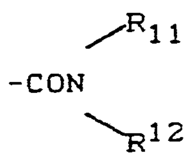 oder 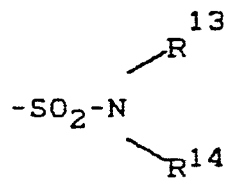 -Rest sein kann

wobei die Reste $R^{11}$ bis $R^{14}$ Wasserstoff, Alkyl mit 1-6 Kohlenstoffatomen oder einen gegebenenfalls substituierten Phenylrest darstellen,

$R^1$, $R^2$, $R^3$ und $R^4$ für Wasserstoff und Alkylgruppen mit 1-3 Kohlenstoffatomen oder für gegebenenfalls durch Halogen, Nitro, Alkyl mit bis zu 3 Kohlenstoffatomen substituierte Phenylreste stehen, sowie

n und m jeweils 0 oder 1 bedeuten,

die Symbole A, B, D und E für $C-R^{15}$ oder bis zu drei dieser Symbole für Stickstoff stehen, wobei

$R^{15}$ für Wasserstoff, Alkyl-, Alkoxy-, Alkylmercapto mit jeweils 1-3 Kohlenstoffatomen, Halogen, Nitro, Trifluormethyl, Cyano, durch $C_1$-$C_4$-Alkyl verestertes Carboxyl, Benzyl oder Phenyl, die jeweils durch $C_1$-$C_3$-Alkyl, Nitro oder Halogen substituiert sein können, steht

und deren physiologisch verträgliche Salze und Prodrug-Formen.

2. 1,8-Verbrückte 4-Chinolon-3-carbonsäuren und Derivate der Formel I gemäß Anspruch 1,

in welcher

$X^1$ für Fluor steht und

$X^2$ Fluor oder Chlor bedeutet.

3. Verfahren zur Herstellung von neuen 1,8-verbrückten 4-Chinolon-3-carbonsäuren und Derivaten der Formel I

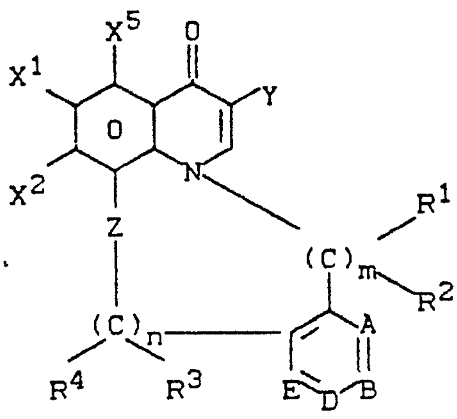

(I)

in welcher

Y eine Carboxylgruppe, eine Nitrilgruppe, eine Estergruppe $-COOR^5$ oder Säureamidgruppe $-CONR^6R^7$ darstellt, wobei $R^5$ für $C_1$-$C_4$-Alkyl steht, $R^6$ und $R^7$ für Wasserstoff oder $C_1$-$C_4$-Alkyl stehen und $R^6$ außerdem gegebenenfalls Phenyl sein kann,

$X^1$ für Wasserstoff, Nitro, Alkyl mit 1-3 Kohlenstoffatomen und Halogen steht,

$X^2$ Halogen, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff oder einen Aminrest $NR^8$ steht, wobei $R^8$ Wasserstoff oder einen gegebenenfalls durch Halogen, Trifluormethyl, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen oder einen gegebenenfalls durch Halogen, eine Nitrogruppe, eine Alkylgruppe mit 1-3 Kohlenstoffatomen substituierten Phenylrest bedeutet, sowie ferner einen Acylrest $R^9CO$- bzw. $R^{10}SO_2$- darstellt, wobei $R^9$ und $R^{10}$ Alkylreste mit 1-6 Kohlenstoffatomen oder gegebenenfalls substituierte Phenylreste darstellen, sowie ein

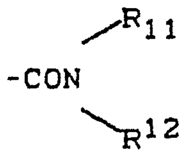 oder 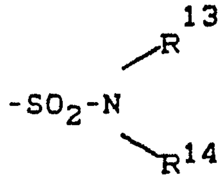 -Rest sein kann

wobei die Reste $R^{11}$ bis $R^{14}$ Wasserstoff, Alkyl mit 1-6 Kohlenstoffatomen oder einen gegebenenfalls substituierten Phenylrest darstellen,

$R^1$, $R^2$, $R^3$ und $R^4$ für Wasserstoff und Alkylgruppen mit 1-3 Kohlenstoffatomen oder für gegebenenfalls durch Halogen, Nitro, Alkyl mit bis zu 3 Kohlenstoffatomen substituierte Phenylreste stehen, sowie

n und m jeweils 0 oder 1 bedeuten,

die Symbole A, B, D und E für C-$R^{15}$ oder bis zu drei dieser Symbole für Stickstoff stehen, wobei

$R^{15}$ für Wasserstoff, Alkyl-, Alkoxy-, Alkylmercapto mit jeweils 1-3 Kohlenstoffatomen, Halogen, Nitro, Trifluormethyl, Cyano, durch $C_1$-$C_4$-Alkyl verestertes Carboxyl, Benzyl oder Phenyl, die jeweils durch $C_1$-$C_3$-Alkyl, Nitro oder Halogen substituiert sein können, steht,

dadurch gekennzeichnet, daß man die Enamine der Formel II

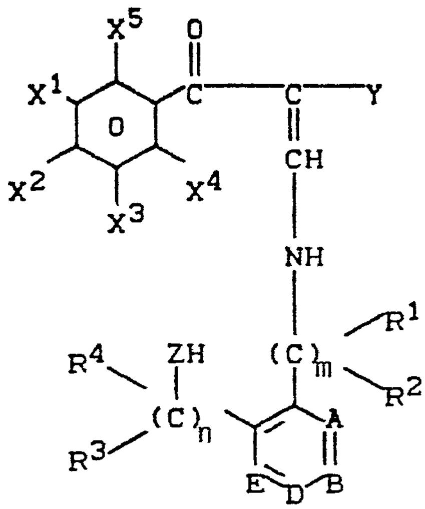

(II)

in welcher

$X^1$, $X^2$, $X^5$, $R^1$-$R^4$, A, B, D, E, Y, Z, m und n die oben angegebene Bedeutung haben und

$X^3$ für Halogen sowie die Nitrogruppe steht,

$X^4$ Halogen, bevorzugt Fluor oder Chlor, eine Nitrogruppe, eine Alkoxy-, Alkylmercapto- oder Alkylsulfonylgruppe mit jeweils 1-3 Kohlenstoffatomen sowie eine Arylsulfonylgruppe bedeutet,

in einer ersten Reaktionsstufe in einem wasserfreien, aprotischen Lösungsmittel in Gegenwart von 1 Äquivalent einer Base zu den 4-Chinolon-3-carbonsäurederivaten der Formel III

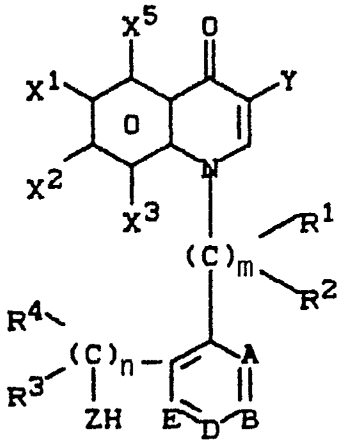

(III)

umsetzt und in einer zweiten Reaktionsstufe in Gegenwart eines weiteren Äquivalents einer Base die Zweitcyclisierung zu den 1,8-verbrückten 4-Chinolon-3-carbonsäurederivaten der Formel I vornimmt, sowie gegebenenfalls die Gruppe Y in an sich bekannter Weise in die Carboxylgruppe und gegebenenfalls diese in ihre Alkali-, Erdalkali- oder Silbersalze überführt.

4. Verfahren nach Anspruch 3, dadurch gekennzeichnet, daß man die Umsetzung der Enamine der Formel II mit 2 Äquivalenten einer Base, ohne Zwischenisolierung der Verbindungen der Formel III, in einer sogenannten Eintopfreaktion zu den 1,8-verbrückten 4-Chinolon-3-carbonsäurederivaten der Formel I durchführt.

5. 1,8-Verbrückte 4-Chinolon-3-carbonsäuren und Derivate der Formel I

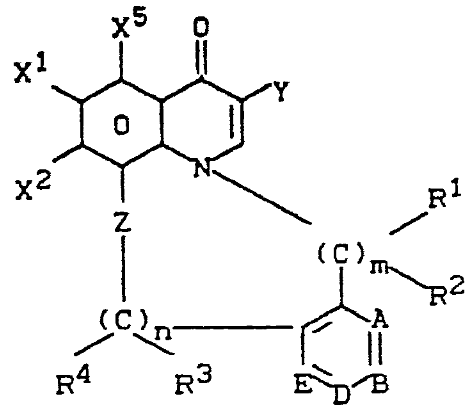

(I)

in welcher

Y eine Carboxylgruppe, eine Nitrilgruppe, eine Estergruppe $-COOR^5$ oder Säureamidgruppe $-CONR^6R^7$ darstellt, wobei $R^5$ für $C_1$-$C_4$-Alkyl steht, $R^6$ und $R^7$ für Wasserstoff oder $C_1$-$C_4$-Alkyl stehen und $R^6$ außerdem gegebenenfalls Phenyl sein kann,

$X^1$ für Wasserstoff, Nitro, Alkyl mit 1-3 Kohlenstoffatomen und Halogen steht,

$X^2$ Halogen, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff oder einen Aminrest $NR^8$ steht, wobei $R^8$ Wasserstoff oder einen gegebenenfalls

durch Halogen, Trifluormethyl, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen oder einen gegebenenfalls durch Halogen, eine Nitrogruppe, eine Alkylgruppe mit 1-3 Kohlenstoffatomen substituierten Phenylrest bedeutet, sowie ferner einen Acylrest $R^9CO$- bzw. $R^{10}SO_2$- darstellt, wobei $R^9$ und $R^{10}$ Alkylreste mit 1-6 Kohlenstoffatomen oder gegebenenfalls substituierte Phenylreste darstellen, sowie ein

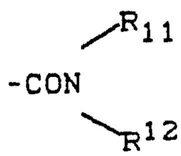 oder 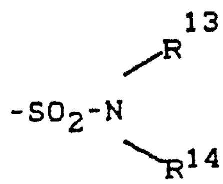 -Rest sein kann

wobei die Reste $R^{11}$ bis $R^{14}$ Wasserstoff, Alkyl mit 1-6 Kohlenstoffatomen oder einen gegebenenfalls substituierten Phenylrest darstellen,

$R^1$, $R^2$, $R^3$ und $R^4$ für Wasserstoff und Alkylgruppen mit 1-3 Kohlenstoffatomen oder für gegebenenfalls durch Halogen, Nitro, Alkyl mit bis zu 3 Kohlenstoffatomen substituierte Phenylreste stehen, sowie

n und m jeweils 0 oder 1 bedeuten,

die Symbole A, B, D und E für $C-R^{15}$ oder bis zu drei dieser Symbole für Stickstoff stehen, wobei

$R^{15}$ für Wasserstoff, Alkyl-, Alkoxy-, Alkylmercapto mit jeweils 1-3 Kohlenstoffatomen, Halogen, Nitro, Trifluormethyl, Cyano, durch $C_1$-$C_4$-Alkyl verestertes Carboxyl, Benzyl oder Phenyl, die jeweils durch $C_1$-$C_3$-Alkyl, Nitro oder Halogen substituiert sein können, steht

und deren physiologisch verträgliche Salze und Prodrug-Formen zur Anwendung in einem Verfahren zur therapeutischen Behandlung des menschlichen oder tierischen Körpers.

6. Arzneimittel, enthaltend 1,8-verbrückte 4-Chinolon-3-carbonsäuren und Derivate der Formel I

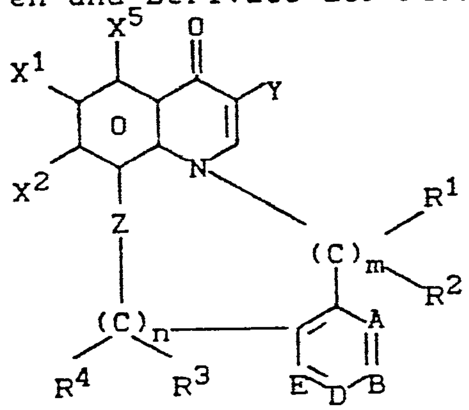

(I)

in welcher

Y eine Carboxylgruppe, eine Nitrilgruppe, eine Estergruppe $-COOR^5$ oder Säureamidgruppe $-CONR^6R^7$ darstellt, wobei $R^5$ für $C_1$-$C_4$-Alkyl steht, $R^6$ und $R^7$ für Wasserstoff oder $C_1$-$C_4$-Alkyl stehen und $R^6$ außerdem gegebenenfalls Phenyl sein kann,

$X^1$ für Wasserstoff, Nitro, Alkyl mit 1-3 Kohlenstoffatomen und Halogen steht,

$X^2$ Halogen, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff oder einen Aminrest $NR^8$ steht, wobei $R^8$ Wasserstoff oder einen gegebenenfalls durch Halogen, Trifluormethyl, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen oder einen gegebenenfalls durch Halogen, eine Nitrogruppe, eine Alkylgruppe mit 1-3 Kohlenstoffatomen substituierten Phenylrest bedeutet, sowie ferner einen Acylrest $R^9CO-$ bzw. $R^{10}SO_2-$ darstellt, wobei $R^9$ und $R^{10}$ Alkylreste mit 1-6 Kohlenstoffatomen oder gegebenenfalls substituierte Phenylreste darstellen, sowie ein

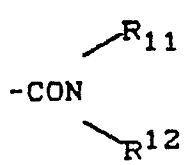 oder 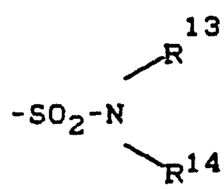 -Rest sein kann

wobei die Reste $R^{11}$ bis $R^{14}$ Wasserstoff, Alkyl mit 1-6 Kohlenstoffatomen oder einen gegebenenfalls substituierten Phenylrest darstellen,

$R^1$, $R^2$, $R^3$ und $R^4$ für Wasserstoff und Alkylgruppen mit 1-3 Kohlenstoffatomen oder für gegebenenfalls durch Halogen, Nitro, Alkyl mit bis zu 3 Kohlenstoffatomen substituierte Phenylreste stehen, sowie

n und m jeweils 0 oder 1 bedeuten,

die Symbole A, B, D und E für C-$R^{15}$ oder bis zu drei dieser Symbole für Stickstoff stehen, wobei

$R^{15}$ für Wasserstoff, Alkyl-, Alkoxy-, Alkylmercapto mit jeweils 1-3 Kohlenstoffatomen, Halogen, Nitro, Trifluormethyl, Cyano, durch $C_1$-$C_4$-Alkyl verestertes Carboxyl, Benzyl oder Phenyl, die jeweils durch $C_1$-$C_3$-Alkyl, Nitro oder Halogen substituiert sein können, steht

und deren physiologisch verträgliche Salze und Prodrug-Formen.

7. Verwendung von 1,8-verbrückten 4-Chinolon-3-carbonsäuren und Derivatender Formel I

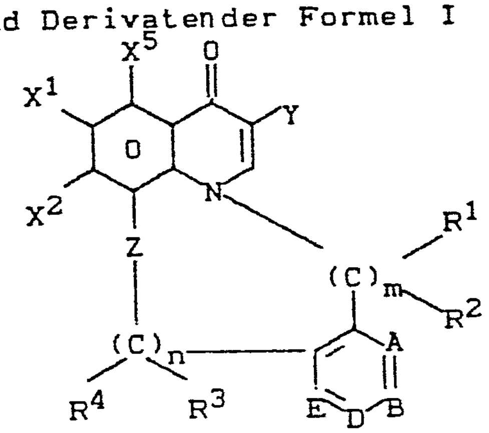

(I)

in welcher

Y eine Carboxylgruppe, eine Nitrilgruppe, eine Estergruppe $-COOR^5$ oder Säureamidgruppe $-CONR^6R^7$ darstellt, wobei $R^5$ für $C_1$-$C_4$-Alkyl steht, $R^6$ und $R^7$ für Wasserstoff oder $C_1$-$C_4$-Alkyl stehen und $R^6$ außerdem gegebenenfalls Phenyl sein kann,

$X^1$ für Wasserstoff, Nitro, Alkyl mit 1-3 Kohlenstoffatomen und Halogen steht,

$X^2$ Halogen, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff oder einen Aminrest $NR^8$ steht, wobei $R^8$ Wasserstoff oder einen gegebenenfalls durch Halogen, Trifluormethyl, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen oder einen gegebenenfalls durch Halogen, eine Nitrogruppe, eine Alkylgruppe mit 1-3 Kohlenstoffatomen substituierten Phenylrest bedeutet, sowie ferner einen Acylrest $R^9CO$- bzw. $R^{10}SO_2$- darstellt, wobei $R^9$ und $R^{10}$ Alkylreste mit 1-6 Kohlenstoffatomen oder gegebenenfalls substituierte Phenylreste darstellen, sowie ein

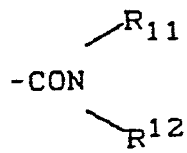 oder 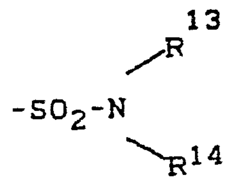 -Rest sein kann

wobei die Reste $R^{11}$ bis $R^{14}$ Wasserstoff, Alkyl mit 1-6 Kohlenstoffatomen oder einen gegebenenfalls substituierte Phenylreste darstellen,

$R^1$, $R^2$, $R^3$ und $R^4$ für Wasserstoff und Alkylgruppen mit 1-3 Kohlenstoffatomen oder für gegebenenfalls durch Halogen, Nitro, Alkyl mit bis zu 3 Kohlenstoffatomen substituierte Phenylreste stehen, sowie

n und m jeweils 0 oder 1 bedeuten,

die Symbole A, B, D und E für C-$R^{15}$ oder bis zu drei dieser Symbole für Stickstoff stehen, wobei

$R^{15}$ für Wasserstoff, Alkyl-, Alkoxy-, Alkylmercapto mit jeweils 1-3 Kohlenstoffatomen, Halogen, Nitro, Trifluormethyl, Cyano, durch $C_1$-$C_4$-Alkyl verestertes Carboxyl, Benzyl oder Phenyl, die jeweils durch $C_1$-$C_3$-Alkyl, Nitro oder Halogen substituiert sein können, steht

und deren physiologisch verträglichen Salzen und Prodrug-Formen zur Herstellung von Arzneimitteln.

8. Tierfutter oder Tierfutterzusätze, enthaltend 1,8-verbrückte 4-Chinolon-3-carbonsäuren und Derivate der Formel I

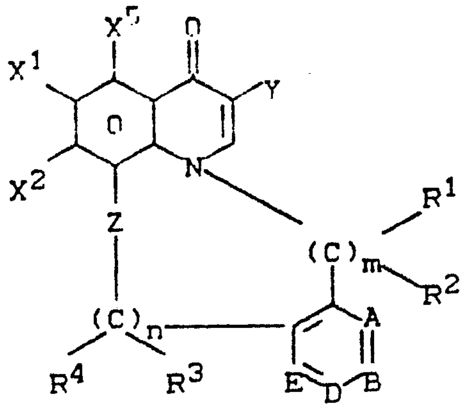

(I)

in welcher

Y eine Carboxylgruppe, eine Nitrilgruppe, eine Estergruppe $-COOR^5$ oder Säureamidgruppe $-CONR^6R^7$ darstellt, wobei $R^5$ für $C_1$-$C_4$-Alkyl steht, $R^6$ und $R^7$ für Wasserstoff oder $C_1$-$C_4$-Alkyl stehen und $R^6$ außerdem gegebenenfalls Phenyl sein kann,

$X^1$ für Wasserstoff, Nitro, Alkyl mit 1-3 Kohlenstoffatomen und Halogen steht,

$X^2$ Halogen, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff oder einen Aminrest $NR^8$ steht, wobei $R^8$ Wasserstoff oder einen gegebenenfalls durch Halogen, Trifluormethyl, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen oder einen gegebenenfalls durch Halogen, eine Nitrogruppe, eine Alkylgruppe mit 1-3 Kohlenstoffatomen substituierten Phenylrest bedeutet, sowie ferner einen Acylrest $R^9CO$- bzw. $R^{10}SO_2$- darstellt, wobei $R^9$ und $R^{10}$ Alkylreste mit 1-6 Kohlenstoffatomen oder gegebenenfalls substituierte Phenylreste darstellen, sowie ein

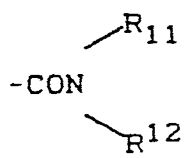 oder 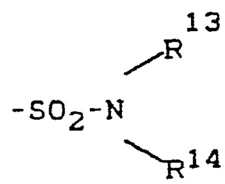 -Rest sein kann

wobei die Reste $R^{11}$ bis $R^{14}$ Wasserstoff, Alkyl mit 1-6 Kohlenstoffatomen oder einen gegebenenfalls substituierten Phenylrest darstellen,

$R^1$, $R^2$, $R^3$ und $R^4$ für Wasserstoff und Alkylgruppen mit 1-3 Kohlenstoffatomen oder für gegebenenfalls durch Halogen, Nitro, Alkyl mit bis zu 3 Kohlenstoffatomen substituierte Phenylreste stehen, sowie

n und m jeweils 0 oder 1 bedeuten,

die Symbole A, B, D und E für C-$R^{15}$ oder bis zu drei dieser Symbole für Stickstoff stehen, wobei

$R^{15}$ für Wasserstoff, Alkyl-, Alkoxy-, Alkylmercapto mit jeweils 1-3 Kohlenstoffatomen, Halogen, Nitro, Trifluormethyl, Cyano, durch $C_1$-$C_4$-Alkyl verestertes Carboxyl, Benzyl oder Phenyl, die jeweils durch $C_1$-$C_3$-Alkyl, Nitro oder Halogen substituiert sein können, steht

oder deren physiologisch verträgliche Salze und Prodrug-Formen.

9. Enamine der Formel II

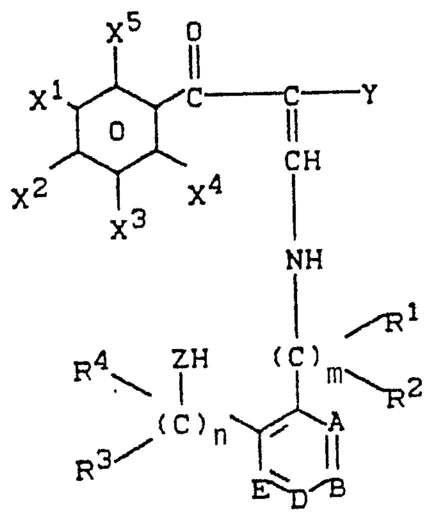

(II)

in welcher

$X^1$, $X^2$, $X^5$, $R^1$-$R^4$, A, B, D, E, Y, Z, m und n die in Anspruch 1 angegebene Bedeutung haben und

$X^3$ für Halogen sowie die Nitrogruppe steht,

$X^4$ Halogen, eine Nitrogruppe, eine Alkoxy-, Alkylmercapto- oder Alkylsulfonylgruppe mit jeweils 1-3 Kohlenstoffatomen sowie eine Arylsulfonylgruppe bedeutet.

10. Verfahren zur Herstellung von Enaminen der Formel II

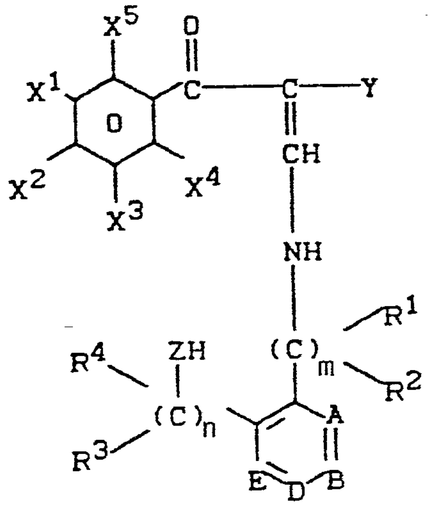

(II)

in welcher

$X^1$, $X^2$, $X^5$, $R^1$-$R^4$, A, B, D, E, Y, Z, m und n die in Anspruch 3 angegebene Bedeutung haben und

$X^3$ für Halogen sowie die Nitrogruppe steht,

$X^4$ Halogen, eine Nitrogruppe, eine Alkoxy-, Alkylmercapto- oder Alkylsulfonylgruppe mit jeweils 1-3 Kohlenstoffatomen sowie eine Arylsulfonylgruppe bedeutet,

dadurch gekennzeichnet, daß man Enolether der Formel IV

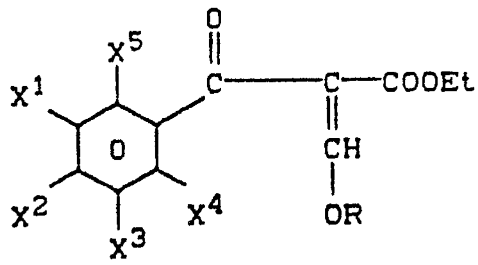

IV

mit primären Aminen der Formel V

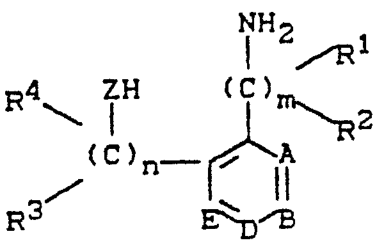

V

umsetzt, wobei die Restedefinition $X^1$ - $X^5$, $R^1$ - $R^4$, Y, Z, A, B, D, E, n und m die in Anspruch 3 angegebene Bedeutung haben und R für $CH_3$, $C_2H_5$ oder $n-C_3H_7$ steht.